Europäisches Patentamt

European Patent Office

Office européen des brevets

(11) Numéro de publication : **0 436 691 B1**

(12) **FASCICULE DE BREVET EUROPEEN**

(45) Date de publication du fascicule du brevet :
15.09.93 Bulletin 93/37

(51) Int. Cl.⁵ : **C07C 69/54,** C07C 67/54,
C07C 67/08

(21) Numéro de dépôt : **90910757.5**

(22) Date de dépôt : **06.07.90**

(86) Numéro de dépôt international :
**PCT/FR90/00516**

(87) Numéro de publication internationale :
**WO 91/01966 21.02.91 Gazette 91/05**

(54) PROCEDE DE PREPARATION EN CONTINU D'ACRYLATES LEGERS.

(30) Priorité : **03.08.89 FR 8910483**

(43) Date de publication de la demande :
**17.07.91 Bulletin 91/29**

(45) Mention de la délivrance du brevet :
**15.09.93 Bulletin 93/37**

(84) Etats contractants désignés :
**AT BE CH DE DK ES FR GB IT LI LU NL SE**

(56) Documents cités :
**GB-A- 2 016 461**

(73) Titulaire : **ELF ATOCHEM S.A.**
**4 & 8, Cours Michelet La Défense 10**
**F-92800 Puteaux (FR)**

(72) Inventeur : **ESCH, Marc**
**23, rue Goethe**
**F-57800 Freyming-Merlebach (FR)**
Inventeur : **COLIN, Nadine**
**32, rue Pierrard**
**F-57500 Saint-Avold (FR)**
Inventeur : **GUENEZ, Dominique**
**13, rue de la Colline Freybouse**
**F-57660 Grostenquin (FR)**
Inventeur : **MARAVAL, Michel**
**12, rue Georges-Brassens**
**F-60700 Pont-Saint-Maxence (FR)**

(74) Mandataire : **Rieux, Michel et al**
**ATOCHEM Département Propriété Industrielle**
**4, Cours Michelet - La Défense 10 - Cedex 42**
**F-92091 Paris-La Défense (FR)**

EP 0 436 691 B1

## Description

La présente invention concerne un procédé de préparation en continu d'acrylates légers à partir d'une solution aqueuse d'acide acrylique et d'un alcool léger. De façon plus particulière, la présente invention concerne un procédé selon lequel on effectue, en phase liquide, la réaction d'estérification d'une solution aqueuse d'acide acrylique par du méthanol ou de l'éthanol de façon à préparer en continu les esters méthyliques ou éthyliques d'acide acrylique.

Il est connu, de façon générale, voir US 4 076 950, de préparer les acrylates légers (de méthyle ou d'éthyle) par estérification d'acide acrylique purifié par un alcool léger. En particulier on connaît par le brevet anglais 2 016 461 un procédé d'estérification mettant en oeuvre un excès d'acide. Il est donc nécessaire, dans une première opération, de purifier la solution aqueuse d'acide acrylique dont on dispose au départ et qui titre au minimum 50% en acide acrylique pur dilué dans une quantité importante d'eau (au maximum 50%) et qui comprend généralement, en outre, dans des proportions plus faibles, des impuretés inhérentes à sa production dont notamment de l'acide acétique et des aldéhydes. La présence d'eau dans l'acide acrylique est en effet un élément défavorable pour l'obtention 'd'une bonne conversion par estérification de l'acide, car elle défavorise la formation de l'ester en déplaçant l'équilibre de la réaction dans le sens de l'hydrolyse ; la conversion d'acide acrylique serait alors si faible que cela nécessiterait d'importants et coûteux recyclages d'acide et d'alcool non convertis. Une méthode classique pour purifier la solution aqueuse d'acide acrylique consiste par exemple à réaliser sur celle-ci une distillation azéotropique extractive, un solvant azéotropique utilisable étant, par exemple, la méthylisobutylcétone. Après traitement de cette solution aqueuse d'acide acrylique dans une installation de purification, on obtient en général un acide acrylique purifié titrant au minimum 98% en acide acrylique pur, contenant une faible quantité d'eau et pouvant dés lors servir de produit de départ pour la réaction d'estérification. Cet acide acrylique purifié est donc, dans une seconde étape, introduit directement dans un réacteur d'estérification qu'on alimente simultanément avec un alcool léger (méthanol ou éthanol). La réaction d'estérification s'effectue à une température comprise entre 50°C et 110°C environ, en présence d'au moins un catalyseur d'estérification et sous une pression égale à la pression atmosphérique ou légèrement supérieure. Les produits réactionnels issus du réacteur, constitués d'un mélange d'acrylate léger, d'eau et de composés de départ n'ayant pas réagi, sont alors envoyés en fond d'une colonne de distillation ; en pied de ladite colonne on récupère un mélange d'acide acrylique, d'eau et d'impuretés lourdes issues de la production d'acide acrylique et/ou de la réaction d'estérification, ce flux étant alors recyclé vers le réacteur d'estérification afin d'assurer, en plus du flux d'acide acrylique provenant du dispositif de purification, l'alimentation dudit réacteur en matière première pour la réaction d'estérification. En tête de ladite colonne on récupère un mélange constitué d'acrylate léger, d'eau et d'alcool qu'on envoie alors vers un dispositif de purification comprenant, dans une première étape, un lavage à l'eau afin de séparer l'acrylate de l'alcool n'ayant pas réagi, suivi d'une distillation de la phase aqueuse résultante, ce afin de récupérer l'alcool qui sera, par la suite, recyclé à la réaction, ainsi que d'une distillation de la phase organique résultante, cette dernière distillation permettant, dans un premier temps, d'éliminer les impuretés légères, puis, dans un second temps, d'éliminer les impuretés lourdes résiduelles, pour aboutir finalement à l'obtention d'une solution d'acrylate titrant au minimum 99% en acrylate pur.

L'inconvénient de cette méthode réside dans la nécessité de disposer d'une unité de purification comprenant des appareillages coûteux et encombrants.

Le problème que la présente invention se propose de résoudre consiste donc à mettre au point un procédé n'ayant pas les inconvénients précédents et permettant de préparer en continu l'acrylate de méthyle ou l'acrylate d'éthyle sans avoir recours à un dispositif.spécifique de purification de la solution aqueuse d'acide acrylique.

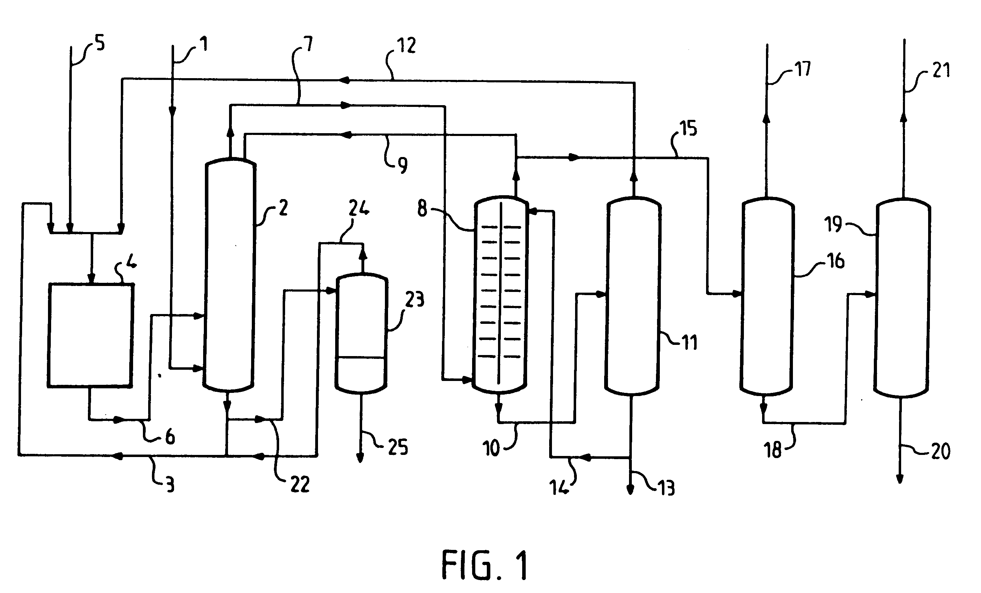

Ce but est atteint selon la présente invention grâce à un nouveau procédé, décrit par référence à la figure 1 en annexe, permettant la préparation en continu d'acrylates légers à partir d'une solution aqueuse d'acide acrylique et d'un alcool léger, la réaction d'estérification s'effectuant en phase liquide dans un réacteur 4, à une température comprise entre 50°C et 110°C environ, en présence d'au moins un catalyseur d'estérification, les produits réactionnels issus du réacteur 4 constitués d'un mélange d'acrylate léger, d'eau et de composés de départ n'ayant pas réagi, étant envoyés en fond d'une colonne de distillation 2 et l'acrylate léger étant récupéré en tête de ladite colonne de distillation 2 et envoyé vers un dispositif de purification, caractérisé en ce que la solution aqueuse d'acide acrylique est introduite en fond de ladite colonne de distillation 2 où elle est soumise à une distillation azéotropique, le flux obtenu en pied de colonne 2, enrichi en acide acrylique, étant alors recyclé vers le réacteur 4 de manière telle qu'à l'entrée dudit réacteur 4, le rapport molaire de l'acide acrylique à l'alcool léger est compris entre 0,5 et 4,5 environ.

La solution aqueuse d'acide acrylique à partir de laquelle on prépare l'acrylate léger comprend de préférence de 50 à 70% d'acide acrylique pur dilué dans une quantité importante d'eau (de 30% à 50% environ) et comprenant, dans des proportions plus faibles, des impuretés inhérentes à sa production. L'originalité du procédé selon l'invention est basée sur le fait que la solution aqueuse d'acide acrylique n'est plus, préalablement à son introduction dans le réacteur d'estérification, purifiée dans une installation spécifique de purification, mais qu'elle est directement introduite, par l'intermédiaire d'une canalisation 1, en fond de la colonne de distillation 2 servant à séparer les produits réactionnels issus du réacteur d'estérification 4, ce qui offre l'avantage de s'affranchir de l'utilisation de solvants azéotropiques tels que, par exemple, la méthylisobutylcétone.

L'alcool de départ (méthanol ou éthanol) qu'on introduit directement dans le réacteur d'estérification 4 par l'intermédiaire d'une canalisation 5 est un produit pouvant contenir une certaine quantité d'eau ; celui-ci sera désigné par le terme d'"alcool frais", ce par opposition à l'alcool recyclé provenant de la section de purification des produits réactionnels issus du réacteur et qui est introduit lui dans le réacteur 4 par l'intermédiaire d'une canalisation 12 ; ces deux flux d'alcool serviront donc à alimenter ledit réacteur en matière première pour la réaction d'estérification. La réaction proprement dite s'effectue en phase liquide, dans au moins un réacteur d'estérification 4 pouvant notamment être de type tubulaire à lit fixe, à une température comprise entre 50°C et 110°C environ et préférentiellement entre 80°C et 100°C ; une température de réaction trop élevée entraînerait la polymérisation de l'acide acrylique alors qu'inversement, une température trop basse rendrait la vitesse de réaction très faible.

La pression à l'intérieur du réacteur 4 est de préférence comprise entre 1 bar et 3 bars.

Comme catalyseurs d'estérification, on peut utiliser des résines cationiques fortement acides, celles-ci permettant d'obtenir des résultats satisfaisants.

De plus, la réaction d'estérification s'effectuera de préférence en présence d'au moins un inhibiteur de polymérisation, ce afin de pallier à une éventuelle polymérisation de l'acide acrylique ; comme inhibiteur convenant parfaitement dans le cadre de la présente invention, on peut citer ceux habituellement utilisés tels que l'hydroquinone ou l'éther monoéthylique d'hydroquinone par exemple.

Le temps de séjour des produits dans le réacteur 4 est de préférence compris entre 1 heure et 5 heures environ.

Les produits réactionnels issus du réacteur d'estérification 4 sont envoyés, par l'intermédiaire d'une canalisation 6, en fond d'une colonne de distillation 2 comprenant au moins 5 plateaux. Celle-ci est donc destinée à accueillir d'une part la solution aqueuse d'acide acrylique et d'autre part les produits réactionnels issus du réacteur, constitués d'un mélange d'acrylate léger, d'eau, de composés de départ n'ayant pas réagi et d'un certain nombre d'impuretés provenant soit de la synthèse de l'acide acrylique, soit de la synthèse de l'ester.

Les produits contenus dans ladite colonne de distillation 2 sont soumis à une distillation azéotropique se basant sur la formation des azéotropes légers eau/acrylate léger et eau/alcool permettant ainsi de concentrer en fond de colonne les composés lourds dont notamment l'acide acrylique et de récupérer en tête de colonne un mélange constitué de composés légers dont notamment l'acrylate léger, l'alcool et l'eau. La température de tête de ladite colonne 2 est de préférence comprise entre 60°C et 95°C environ ; de la température de pied, comprise entre 90°C et 120°C environ, dépend notamment l'épuisement des composés légers, donc le taux de transformation à la réaction ainsi que la formation des composés lourds.

Cette distillation azéotropique permet donc d'une part de débarrasser l'ester brut, c'est-à-dire l'ester obtenu directement après la réaction d'estérification et qui n'a été soumis à aucune étape de purification, de l'acide acrylique qu'il contient, et d'autre part de soutirer de l'eau du réacteur 4, favorisant ainsi la réaction d'estérification dans ce dernier. De plus, pour favoriser la distillation de l'eau dans ladite colonne 2, et éviter la distillation de l'acide, il est nécessaire d'envoyer en tête de cette colonne un reflux d'acrylate léger lavé, c'est-à-dire d'acrylate léger débarrassé d'eau et d'alcool ; ce reflux d'acrylate léger lavé provient d'une colonne de lavage 8 recevant à sa base les produits distillés en tête de ladite colonne 2 c'est-à-dire essentiellement un mélange composé d'acrylate léger, d'alcool n'ayant pas réagi et d'eau provenant soit de la réaction d'estérification, soit de la solution aqueuse d'acide acrylique alimentée sur ladite colonne 2, et dont la fonction est de laver l'acrylate léger c'est-à-dire de le débarrasser d'une grande partie de l'eau et de l'alcool qu'il contient, récupérant ainsi en tête de ladite colonne de lavage 8 l'acrylate léger lavé dont une partie est refluée, par l'intermédiaire d'une canalisation 9, en tête de colonne de distillation 2. Le taux de reflux d'acrylate léger lavé envoyé en tête de ladite colonne 2 pour favoriser la formation des azéotropes combinés ester/eau et alcool/eau, est ajusté en fonction de la quantité d'eau présente dans ladite colonne ; selon la présente invention, ce taux sera de préférence compris entre 0,2 et 4 environ.

L'acide acrylique concentré en fond de ladite colonne de distillation 2 titre environ 50% en poids en acide acrylique pur, de 8% à 20% environ en poids d'eau, la partie restante étant constituée essentiellement d'impuretés lourdes issues de la production d'acide acrylique ou de la réaction d'estérification ; une partie de ce flux est amenée au réacteur 4 par l'intermédiaire d'une canalisation 3, la quantité restante étant acheminée, par l'intermédiaire d'une canalisation 22, vers une installation comprenant une colonne de séparation 23 et un évaporateur au sens usuel du terme, les sous-produits lourds étant soutirés en fond de l'évaporateur par l'intermédiaire d'une canalisation 25 et l'acide acrylique récupéré en tête de ladite colonne 23, débarrassé de ses impuretés lourdes, étant amené au réacteur 4 par l'intermédiaire d'une canalisation 24 reliée à la canalisation 3. Ainsi, l'acide acrylique servant de matière première à la réaction d'estérification provient uniquement du pied de la colonne de distillation 2 et il n'y a, par conséquent, aucune alimentation directe au niveau du réacteur d'estérification 4. Le taux de recyclage qui correspond au rapport du débit d'acide acrylique alimenté à la colonne de distillation 2 et qu'on désignera par "acide acrylique frais", sur le débit d'acide acrylique recyclé au réacteur 4 par l'intermédiaire de la canalisation 3 est de préférence compris entre 0,1 et 0,25.

Le flux d'acide acrylique amené au réacteur 4 par la canalisation 3 est tel qu'à l'entrée dudit réacteur, le rapport molaire de l'acide acrylique à l'alcool léger total introduit dans le réacteur 4 est compris entre 0,5 et 4,5 environ; cependant le fait de fonctionner en excès d'acide à l'entrée du réacteur 4 permet d'atteindre une température de réaction plus élevée, et conduit par conséquent à de meilleurs rendements. Par alcool léger total introduit dans le réacteur 4, on entend la somme de l'alcool recyclé provenant de la section de purification des produits réactionnels, introduit dans ledit réacteur 4 par l'intermédiaire de la canalisation 12, et de l'alcool frais directement introduit dans ledit réacteur 4 par l'intermédiaire de la canalisation 5. De plus, l'alimentation de l'installation en réactifs frais est telle que le rapport molaire d'acide acrylique frais sur l'alcool frais est de préférence compris entre 0,5 et 1.

Le mélange, constitué principalement d'acrylate léger, d'alcool n'ayant pas réagi et d'eau, récupéré en tête de la colonne de distillation 2 est envoyé, par l'intermédiaire d'une canalisation 7, en pied d'une colonne de lavage 8 servant à débarrasser l'acrylate léger d'une grande partie d'eau et d'alcool qu'il contient ; en fond de ladite colonne 8, on récupère un flux constitué d'un mélange eau-alcool qu'on envoie, par l'intermédiaire d'une canalisation 10, sur une colonne de récupération d'alcool 11 qui permet de séparer l'alcool de l'eau, l'alcool étant récupéré en tête de ladite colonne 11 puis recyclé au réacteur 4 par l'intermédiaire d'une canalisation 12, l'eau étant concentrée en fond de ladite colonne 11 pour être en partie refluée, par l'intermédiaire d'une canalisation 14, en tête de la colonne 8, la partie restante étant soutirée de l'installation par l'intermédiaire d'une canalisation 13.

En tête de ladite colonne de lavage 8, on récupère un acrylate léger lavé mais contenant encore, comme impuretés, des sous-produits réactionnels ; une partie de ce flux d'acrylate léger lavé est alors refluée en tête de la colonne de distillation 2, la quantité restante étant amenée, par l'intermédiaire d'une canalisation 15, sur une colonne de séparation 16 permettant de débarrasser l'acrylate des sous-produits légers qu'il contient, dont notamment le formol, l'acroléine, l'acétone et les acétates, ceux-ci étant soutirés en tête de ladite colonne 16 par l'intermédiaire d'une canalisation 17. En pied de ladite colonne 16, on récupère l'acrylate léger comportant encore des impuretés lourdes, dont notamment l'acide maléique, l'acide acétique, et les dimères issus de la synthèse de l'acide acrylique ainsi que des composés issus de la réaction d'estérification tels que notamment l'alkoxypropionate d'alkyle, l'acryloxypropionate d'alkyle et le maléate d'alkyle, le groupement alkyle étant soit un groupement méthyle, soit un groupement éthyle ; ce flux soutiré en pied de la colonne 16 est alors amené, par l'intermédiaire d'une canalisation 18, sur une colonne de séparation 19. En pied de ladite colonne 19, on récupère les sous-produits lourds qu'on soutire par l'intermédiaire d'une canalisation 20, l'acrylate léger titrant au moins 99% en poids d'acrylate pur étant récupéré en tête de ladite colonne 19, puis soutiré par l'intermédiaire d'une canalisation 21.

Les exemples non limitatifs suivants sont donnés pour mieux illustrer l'invention :

Exemple 1 :

On considère une installation (telle que celle représentée schématiquement sur la figure 1) de fabrication d'acrylate d'éthyle obtenu par réaction d'estérification en présence d'un catalyseur d'estérification, à une température de 80°C, entre un acide acrylique et de l'éthanol. La pression à l'intérieur du réacteur d'estérification est de 2 bars.

Le tableau I ci-dessous donne les débits des principaux flux de matière à différents niveaux de l'installation, ainsi que leur composition en pourcentages en poids. On désignera par:
- F1 :   le flux d'éthanol frais introduit dans le réacteur 4 par l'intermédiaire de la canalisation 5.
- F2 :   la solution aqueuse d'acide acrylique amenée sur la colonne 2 par l'intermédiaire de la canalisation 1.
- F3 :   le flux d'acide acrylique recyclé au réacteur 4 par l'intermédiaire d'une canalisation 3.
- F4 :   le flux de sous-produits lourds soutiré de l'évaporateur par l'intermédiaire de la canalisation 25.
- F5 :   le flux de produits de distillation sortant en tête de la colonne de distillation 2 et amené sur la colonne de séparation 8 par l'intermédiaire de la canalisation 7.
- F6 :   le flux d'acrylate d'éthyle débarrassé d'eau et d'alcool sortant en tête de la colonne d'extraction 8 et recyclé en tête de la colonne de distillation 2 par l'intermédiaire de la canalisation 9.

4

- F7 : le flux d'eau soutiré en fond de la colonne de séparation 11 par l'intermédiaire de la canalisation 13.
- F8 : le flux d'éthanol recyclé au réacteur 4 par l'intermédiaire de la canalisation 12.
- F9 : le flux de sous-produits légers soutiré en tête de la colonne de séparation 16 par l'intermédiaire de la canalisation 17.
- F10 : le flux d'acrylate d'éthyle pur obtenu en tête de la colonne de séparation 19 et soutiré par l'intermédiaire de la canalisation 21.

Le rendement global de l'atelier obtenu dans ce cas de figure, exprimé en moles d'ester produit par moles d'acide introduit est de 97,6%.

Tableau I

| Flux | Débit en kg/h | Composition (% en poids) | | | | | |
| --- | --- | --- | --- | --- | --- | --- | --- |
| | | Acide acrylique | Acrylate d'éthyle | Alcool | Eau | Somme des composés légers | Somme des composés lourds |
| F1 | 2037 | - | - | 94 | 6 | - | - |
| F2 | 5550 | 58 | - | - | 34 | 2,28 | 5,72 |
| F3 | 22800 | 45,12 | 1,79 | 3,17 | 16,62 | 1,04 | 32,36 |
| F4 | 200 | 14,7 | 0,8 | 0,6 | 0,5 | - | 83,4 |
| F5 | 22800 | - | 66,78 | 18,34 | 12,18 | 2,83 | 0,17 |
| F6 | 4800 | - | 92,35 | - | 2,6 | 4,95 | 0,1 |
| F7 | 1800 | 2,3 | 0,005 | 0,005 | 97,69 | - | - |
| F8 | 7875 | - | 14,84 | 74,26 | 8,69 | 2,12 | 0,09 |
| F9 | 260 | - | 42 | 0,37 | 3,2 | 54,43 | - |
| F10 | 4370 | - | 99,93 | 0,0063 | 0,0027 | 0,061 | - |

Exemple 2 :

On considère une installation (telle que celle représentée schématiquement sur la figure 1) de fabrication d'acrylate de méthyle obtenu par réaction d'estérification, à une température de 85°C, entre un acide acrylique et du méthanol, en présence de 1,2 litre de résines jouant le rôle de catalyseur d'estérification. Le temps de séjour des produits à l'intérieur du réacteur est de 95 minutes. La température de tête de colonne de distillation 2 est de 64°C, la température du bouilleur de 98°C et la pression à l'intérieur de ladite colonne 2 de 600 mm

6

Hg ($8 \times 10^4$ Pa).

Le tableau II ci-dessous donne les débits des principaux flux de matière à différents niveaux de l'installation ainsi que leur composition en pourcentage en poids.

Les définitions des flux F1, F2, F3, F5 et F6 sont celles données dans l'exemple 1 précédent.

Le rendement global de l'essai obtenu dans ce cas de figure, exprimé en moles d'ester produit par moles d'acide introduit, est de 98,7%.

Tableau II

| Flux | Débit en g/h | Composition (% en poids) | | | | | |
|------|------|------|------|------|------|------|------|
| | | Acide acrylique | Acrylate de méthyle | Alcool | Eau | Somme des composés légers | Somme des composés lourds |
| F1 | 48,4 | – | – | 100 | – | – | – |
| F2 | 104,0 | 64,89 | – | – | 32,10 | 2,7 | 0,31 |
| F3 | 699 | 63,31 | 0,1 | 0,33 | 18,25 | 1,38 | 16,63 |
| F5 | 647,3 | – | 90,11 | 1,58 | 7,67 | 0,6 | 0,04 |
| F6 | 505,9 | – | 99,57 | – | 0,01 | 0,01 | 0,41 |

EP 0 436 691 B1

## Revendications

1. Procédé de préparation en continu d'acrylates légers à partir d'une solution aqueuse d'acide acrylique et d'un alcool léger, la réaction d'estérification s'effectuant en phase liquide dans un réacteur (4), à une température comprise entre 50 et 110°C, en présence d'au moins un catalyseur d'estérification, les produits réactionnels issus du réacteur (4), constitués d'un mélange d'acrylate léger, d'eau et de composés de départ n'ayant pas réagi, étant envoyés en fond d'une colonne de distillation (2) et l'acrylate léger étant récupéré en tête de ladite colonne (2) et envoyé vers un dispositif de purification, caractérisé en ce que la solution aqueuse d'acide acrylique est introduite en fond de ladite colonne de distillation (2) où elle est soumise à une distillation azéotropique, le flux obtenu en pied de colonne (2), enrichi en acide acrylique, étant alors recyclé vers le réacteur (4) de manière telle qu'à l'entrée dudit réacteur (4), le rapport molaire de l'acide acrylique à l'alcool léger est compris entre 0,5 et 4,5, l'acrylate léger étant choisi parmi l'acrylate de méthyle et l'acrylate d'éthyle et l'alcool léger étant choisi parmi le méthanol et l'éthanol.

2. Procédé selon la revendication 1 caractérisé en ce que la solution aqueuse d'acide acrylique comprend de 50 à 70% en poids d'acide acrylique pur.

3. Procédé selon l'une des revendications 1 et 2 caractérisé en ce que la distillation s'effectuant dans la colonne (2) est azéotropique.

4. Procédé selon l'une des revendications 1 à 3 caractérisé en ce que le taux d'acrylate léger lavé reflué en tête de la colonne (2) est compris entre 0,2 et 4.

5. Procédé selon l'une des revendications 1 à 4 caractérisé en ce que le réacteur (4) ne reçoit pas d'autre alimentation en acide acrylique que le flux provenant du pied de la colonne (2).

6. Procédé selon l'une des revendications 1 à 5 caractérisé en ce que le rapport d'acide acrylique alimenté à la colonne (2) sur l'acide acrylique recyclé au réacteur (4) est compris entre 0,1 et 0,25.

7. Procédé selon l'une des revendications 1 à 6 caractérisé en ce que le rapport molaire d'acide acrylique frais sur l'alcool frais est compris entre 0,5 et 1.

## Patentansprüche

1. Verfahren zur kontinuierlichen Herstellung von leichten Acrylaten aus einer wäßrigen Acrylsäurelösung und einem leichten Alkohol, wobei die Veresteningsreaktion in flüssiger Phase in einem Reaktor (4) bei einer Temperatur zwischen 50° und 110°C in Gegenwart mindestens eines Veresterungskatalysators erfolgt, die aus dem Reaktor (4) austretenden Reaktionsprodukte, die aus einer Mischung aus leichtem Acrylat, Wasser und nicht ausreagierten Ausgangsverbindungen bestehen, in den Boden einer Destillationskolonne (2) geführt werden und das leichte Acrylat am Kopf der genannten Kolonne (2) gewonnen und zu einer Reinigungseinrichtung geführt wird, dadurch gekennzeichnet, daß die wäßrige Acrylsäurelösung in den Boden der genannten Destillationskolonne (2) eingebracht wird, wo sie einer azeotropischen Destillation unterworfen wird, wobei dann der am Fuß der Kolonne (2) erhaltene, mit Acrylsäure angereicherte Strom derart zum genannten Reaktor (4) rückgeführt wird, daß am Eingang des genannten Reaktors (4) das Molverhältnis der Acrylsäure zum leichten Alkohol zwischen 0,5 und 4,5 beträgt, wobei als leichtes Acrylat Methylacrylat oder Ethylacrylat und als leichter Alkohol Methanol oder Ethanol gewählt wird.

2. Verfahren nach Anspruch 1, dadurch gekennzeichnet, daß die wäßrige Acrylsäurelösung 50 bis 70 Gew.-% reine Acrylsäure enthält.

3. Verfahren nach Anspruch 1 oder 2, dadurch gekennzeichnet, daß die in der Kolonne (2) erfolgende Destillation azeotropisch ist.

4. Verfahren nach einem der Ansprüche 1 bis 3, dadurch gekennzeichnet, daß der Prozentsatz des gewaschenen, zum Kopf der Kolonne (2) zurückströmenden leichten Acrylats zwischen 0,2 und 4 beträgt.

5. Verfahren nach einem der Ansprüche 1 bis 4, dadurch gekennzeichnet, daß der Reaktor (4) keine andere Anspeisung an Acrylsäure als den vom Fuß der Kolonne (2) kommenden Strom erhält.

6. Verfahren nach einem der Ansprüche 1 bis 5, dadurch gekennzeichnet, daß das Verhältnis der der Kolonne (2) zugeführten Acrylsäure zu der zum Reaktor (4) rückgeführten Acrylsäure zwischen 0,1 und 0,25 beträgt.

7. Verfahren nach einem der Ansprüche 1 bis 6, dadurch gekennzeichnet, daß das Molverhältnis der frischen Acrylsäure zum frischen Alkohol zwischen 0,5 und 1 beträgt.

## Claims

1. Process for the continuous preparation of lower acrylates from an aqueous solution of acrylic acid and from a lower alcohol, the esterification reaction taking place in liquid phase in a reactor (4), at a temperature of between 50°C and 110°C, in the presence of at least one esterification catalyst, the reaction products leaving the reactor (4), consisting of a mixture of lower acrylate, water and unreacted starting compounds, being sent to the bottom of a distillation column (2) and the lower acrylate being recovered at the head of the said column (2) and sent towards a purification device, characterised in that the aqueous solution of acrylic acid is introduced at the bottom of the said distillation column (2) where it is subjected to an azeotropic distillation, the stream obtained at the foot of the column (2), enriched in acrylic acid, then being recycled towards the reactor (4) so that at the entry of the said reactor (4) the molar ratio of acrylic acid to the lower alcohol is between 0.5 and 4.5, the lower acrylate being chosen from methyl acrylate and ethyl acrylate and the lower alcohol being chosen from methanol and ethanol.

2. Process according to Claim 1, characterised in that the aqueous solution of acrylic acid contains from 50 to 70 % by weight of pure acrylic acid.

3. Process according to either of Claims 1 and 2, characterised in that the distillation taking place in the column (2) is azeotropic.

4. Process according to one of Claims 1 to 3, characterised in that the proportion of washed lower acrylate refluxed at the head of the column (2) is between 0.2 and 4.

5. Process according to one of Claims 1 to 4, characterised in that the reactor (4) does not receive an acrylic acid feed other than the stream originating from the foot of the column (2).

6. Process according to one of Claims 1 to 5, characterised in that the ratio of acrylic acid fed to the column (2) to the acrylic acid recycled to the reactor (4) is between 0.1 and 0.25.

7. Process according to one of Claims 1 to 6, characterised in that the molar ratio of fresh acrylic acid to the fresh alcohol is between 0.5 and 1.

FIG. 1

EP 0 436 691 B1